# EUROPEAN PATENT APPLICATION

(11) **EP 2 058 313 A2**
(43) Date of publication of application: **13.05.2009**
(21) Application number: 08017172.1
(22) Date of filing: 30.09.2008
(51) Int. Cl.: C07D 487/04, A61K 31/495, A61K 31/50, A61P 25/20

(54) **Process for synthesis and purification of anhydrous crystalline S-zopiclone**

(30) Priority: 11.10.2007 ES 200702675
(71) Applicant: Apotecnia , S.A., 30588 Murcia (ES)
(72) Inventor: Bayod Jasanaba, Miguel-Santos, 30588 Murcia (ES); Ribas Bueno, Cristina, 30588 Murcia (ES)
(74) Representative: Isern-Jara, Nuria

(57) **Abstract**

Process for synthesis and purification of anhydrous crystalline S-zopiclone for the preparation of enantiomerically pure {S)-5-{chloromethyloxycarbonyloxy)-6-{5-chloropyrid-2-yl)-7-oxo-5,6-dihydropyrrolo[3,4b]pyrazine (S)-(I).

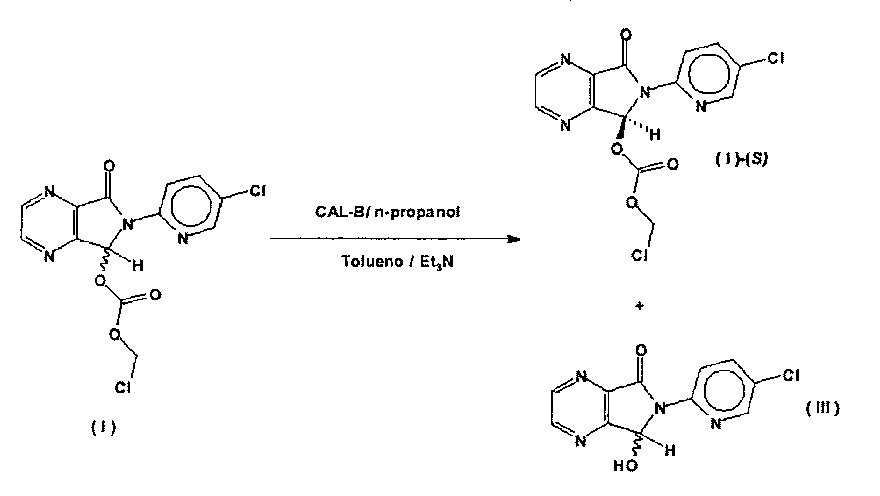

## Description

### Field of the invention

The present invention relates to a new procedure for the synthesis of 5-(chloromethyloxycarbonyloxy)-6-(5-chloropyrid-2-yl)-7-oxo-5,6-dihydropyrrolo[3,4b]pyrazine (S)-(I) (CAS Registry Number 508169-20-8) and to its conversion in (S)-zopiclone (S)-(II) (CAS Registry Number 138729-47-2), which is purified by column chromatography and isolated as an anhydrous crystalline polymorph.

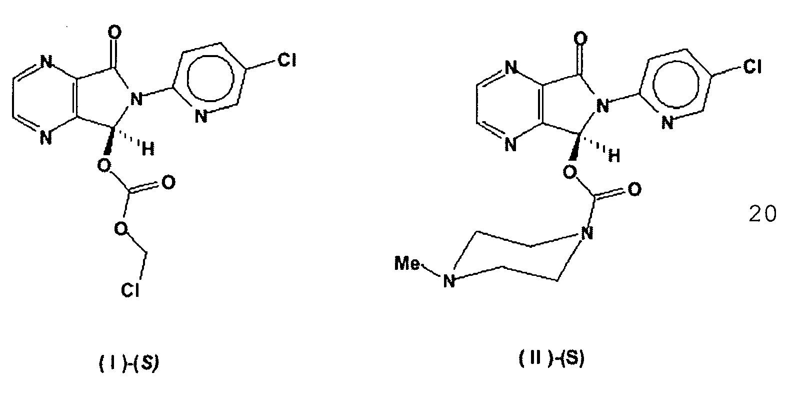

### Short description of the drawings

| | |
|---|---|
| Figure 1 | Scheme for the synthesis of racemic zopiclone |
| Figure 2 | Synthesis of precursors of (S)-zopiclone by enzymatic resolutions |
| Figure 3 | Synthesis of (S)-5-(chloromethyloxycarbonyloxy)-6-(5-chloropyrid-2-yl)-7-oxo-5,6-dihydropyrrolo-[3,4b]pyrazine |
| Figure 4 | Impurities of (S)-zopiclone |
| Figure 5 | Chromatography of S)-zopiclone |
| Figure 6 | Crystals of (S)-zopiclone |
| Figure 7 | DSC and TG of (S)-zopiclone |
| Figure 8 | Dust X ray diffraction profiles of (S)-zopiclone |

### Background of the invention

The zopiclone is a cyclopyrrolone with hypnotic properties. The zopiclone molecule has a chiral centre. It's a known fact (Chirality 5, 419, 1993) that the dextrorotatory enantiomer (S)-(II) is about twofold more active than the racemic one, whereas the levorotatory isomer is almost inactive. Furthermore, according to patent EP 609210-B1, the levorotatory isomer is responsible for the most adverse effects, which are associated with the administration of such medicament.

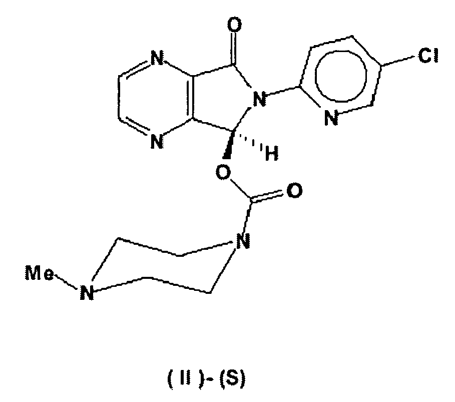

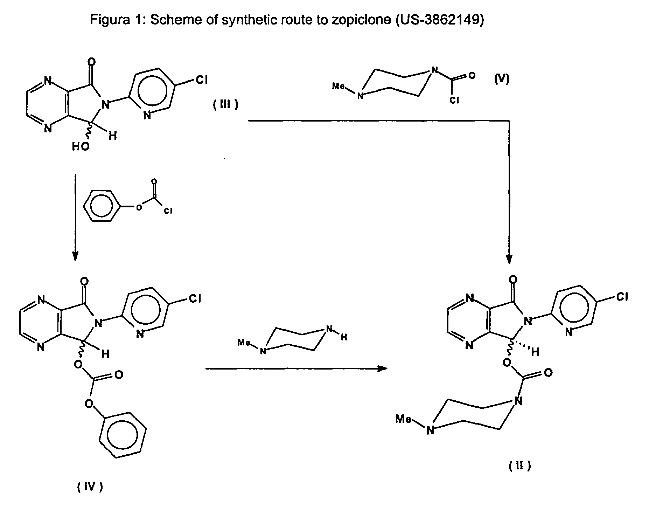

In figure 1 an abstract is enclosed of patent US-3862 149 contents. In such figure is given for the first time a synthetic description of racemic zopiclone (II), through addition of N-methylpiperazine to carbonate of formula (IV), which in turn is obtained through reaction of alcohol of formula (III) with phenyl chloroformiate. It discloses also the synthesis of racemic zopiclone by reaction of alcohol (III) with 1-chlorocarbonyl-4-methylpiperazine (V).

According to patent EP 609210-B1 and related documents the zopiclone enantiomers can be separated through fractional crystallization of diastereoisomeric salts formed with an optically active acid. As optically active acids can be used, for instance, the (+)- or (-)-malic acid (Chirality 5, 419, 1993) or the (+)-O,O'-dibenzoyltartaric acid. In this document the (S)-zopiclone is crystallized from acetonitrile, although the obtained polymorph is not further characterized, neither from crystallographic point of view, nor from results of any thermogravimetric assay.

The patents, which are related to the present one, are:
US20020193378A1 JP06504548T2 W09212980A1 ES2071486T3
US20050043311A1
US20060194806A1
US6319926
US6319926B1
US6444673
US6444673B1
US6864257
US7125874

WO 2007/083188 describes a similar technology, but in this case an acid selected from the group consisting of D-lactic, D-tartaric, D-malic and di-p-tolyl-tartaric acids is used as optically active acid. The product is isolated through concentration to dryness of a solution in methylene chloride, followed by a wash with acetone. In the same document the (S)-zopiclone is prepared by alkalinization of an aqueous suspension of a precursor salt, followed by the crystallization from acetone/acetic anhydride.

In US 2007/0054914 the optically active acid used for the resolution is di-p-tolyl-D-tartaric acid. The product is isolated by concentration to dryness of a solution in methylene chloride, followed by recrystallization from acetonitrile or suspension in isopropanol.

In US 2007/0203145 and WO 2007/088073 the chiral acids used are tartaric acid or ditolyltartaric acid.

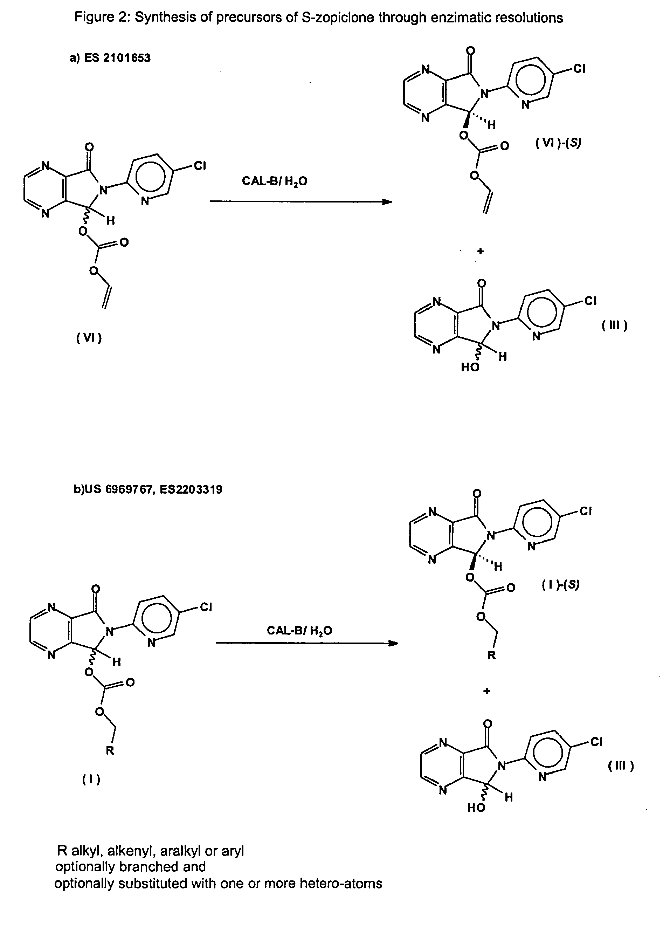

In none of these patent applications is to be found the characterization of obtained polymorph with data from the thermogravimetric assays and only in WO 2007/083188 a dust X ray diffractogram (figure 2 in mentioned document) is given, neither peaks nor parameters of crystal cell being listed.

The enantiomers can also be separated by chromatography on the chiral stationary phase. We can find examples of that technology in Journal of Chromatography 572, 195-202, 1991; Journal of Chromatography A, 1996, 729, 19-28, Journal of Chromatography, Biomedical Applications 617, 271-278, 1993; Journal of Pharmaceutical and Biomedical Analysis 14, 1367-1370, 1996. In WO 2006/136866 a process is described for resolution of zopiclone using Chiralcel^{®} OD, Chiralpak^{®} OD or Chiralpak^{®} AD as a stationary phase.

Another form of carrying out the preparation of (S)-zopiclone is the enzymatic resolution of carbonates of formula (I) obtained from a known product (III). In figure 2 the synthetic procedures for such methodology are abstracted.

As an enzymatic catalyst the *Candida antarctica* lipases are used. These enzymes can be either in free form or in immobilized form, both in commercial preparations or in other contents to this end. It is known that activity and stability of enzymes can be modified by the immobilization or other known treatments, one can profit from that fact to improve process. Preferably a form of immobilization is employed, which is compatible with the high concentrations of organic co-solvent required for increasing the substrate solubility in the medium. The enzyme, which can be immobilized or not, is located preferably in suspension, once the reaction is completed.

In Tetrahedron: Asymm. 8, 995, 1997, and in patent ES-2101653 the enzyme catalyses the reaction between vinyl carbonate (VI) and one molecule of water, in some anhydrous organic solvents. After the preparation of enantiopure carbonate (S)-(VI), this is transformed into (+)-zopiclone by reaction with N-methylpiperazine. The yield of that last synthetic step is rather low, this along with the high cost of vinyl chloroformiate makes the process no longer feasible from an industrial point of view.

In US 6969767, ES-2203319, Tetrahedron Asymmetry 13, 2577-2582, 2002 and Tetrahedron Asymmetry 14, 429-438, 2003, the cases of the art technology are further disclosed, and for the first time the new compounds of formula (I) are described, both in racemic form and in enantiomerically enriched form, with R being alkyl, alkenyl, aralkyl, or aryl, optionally branched and optionally substituted with one or more hetero-atoms.

Preferably the enzymatic resolution is carried out in an anhydrous organic solvent, to which the suitable amount of water is preferably added.

An unexpected aspect of processing is that compound (R)-(III), which is formed during the enzymatic resolution, is spontaneously racemiced in the working conditions, so that after purification, it can be used directly in the preparation of additional racemic carbonate of formula (I). In this way it's possible to make the most of the starting materials and to reduce the amount of residual materials, the latter is a very important factor when the objetive is the industrial application of the process.

Once the respective optically enriched carbonate of formula (S)-(I) is obtained, it is transformed into (S)-zopiclone of formula (S)-(II) through reaction with N-methylpiperazine in an inert organic solvent, for instance acetonitrile, tetrahydrofurane, dioxane or acetone, among others. The procedure is similar to that shown in figure 1.

In Eur. Cryst. Meeting 8, 32, 1983, Mol. Pharmacol. 31, 334, 1987, and J. Chem. Soc. Perkin Trans. 2, 283, 1990, are described the known crystalline polymorphs of zopiclone; a polymorph dihydrate which is racemic, an anhydrous racemic polymorph, which is crystallized in the rhombic system and an anhydrous racemic polymorph, which is crystallized in the monoclinic system. In Chem. Commun. 2204-2205, 2001, are also shown the crystals of anhydrous rhombic polymorph, although they are enantiomerically pure, both the (R) and the (S).

### Abstract of the invention

The present invention describes a new process for the preparation of (S)-5(chloromethyloxycarbonyloxy)-6-(5-chloropyrid-2-il)-7-oxo-5,6-dihidropyrrolo[3,4b]pyrazine, compound of formula (S)-(I), (CAS Registry Number 508169-20-8), which is a key intermediate for the preparation of (S)-zopiclone. The synthetic procedure includes the enzymatic resolution by a lipase of a stereoisomer mixture of (±)-5-(chloromethyloxycarbonyloxy)-6-(5-chloropyrid-2-yl)-7-oxo-5,6-dihydropyrrolo-[3,4b]pyrazine of formula (I) in the presence of n-propanol as a nucleophile.

The present invention also provides a new procedure for the preparation and purification of (S)-zopiclone, compound of formula (S)-(II), starting from (S)-5-(chloromethyloxycarbonyloxy)-6-(5-chloropyrid-2-il)-7-oxo-5,6-dihydropyrrolo-[3,4b]pyrazine, the compound of formula (S)-(I).

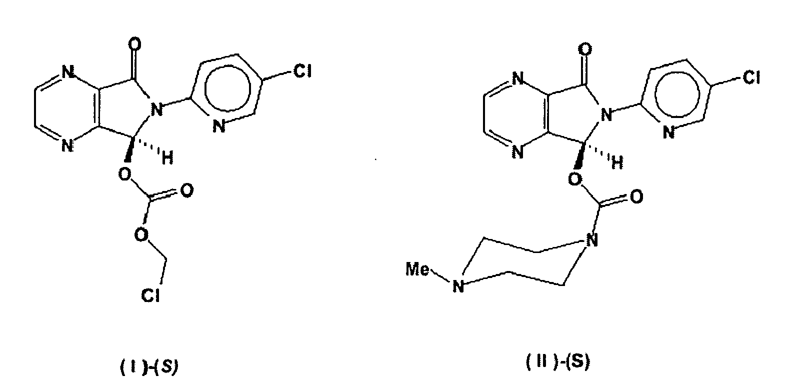

The purification of (S)-zopiclone is carried out by means of column chromatography, followed by an isolation through crystallization from acetone-isopropanol, solvents of class 3 according to the Guideline for Residual Solvents ICH Q3C, instead of using of acetonitrile, a solvent of class 2 according to the above mentioned classification.

The procedures of the present invention have been designed for the fulfilment on an industrial scale, avoiding the limitations of the previous processes.

According to the procedures of the present invention obtained (S)-zopiclone shows a degree of purity, which allows its use as a starting material for the formulation of drugs to be sold in markets ruled by quality criteria such as GMP.

### Detailed description of the invention

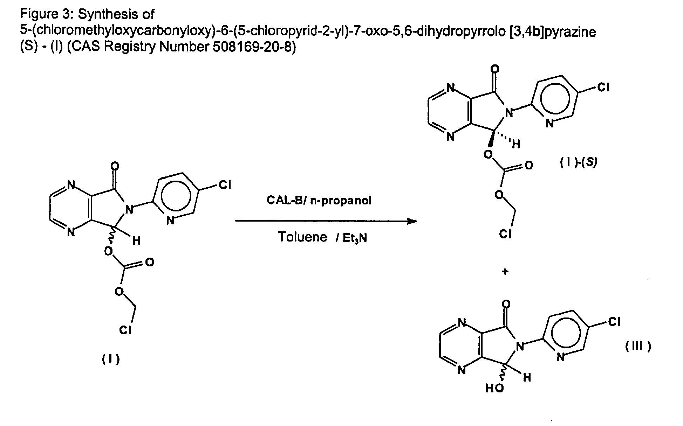

The present invention provides a new process for the preparation of enantiomerically pure (S)-5-(chloromethyloxycarbonyloxy)-6-(5-chloropyrid-2-yl)-7-oxo-5,6-dihydropyrrolo-[3,4b]pyrazine (S)-(I) (CAS Registry Number 508169-20-8) according to the scheme shown in figure 3.

The process can be considered as a stereospecific alcoholysis of a enantiomeric mixture of compound of formula (I) in the presence of an enzyme and an aliphatic amine in an organic solvent. An enantiomer mixture is understood as a racemic mixture or a mixture, which is enriched in (S)-enantiomer. From the GMP manufacturing point of view, the last possibility affords the capability of reprocessing lots with an enantiomeric excess of less than 98 % by the same manufacturing procedure used when the racemic mixture is the starting material, the only remaining requirement is the adjustment of the stoichiometry of the other reagents (lipase, triethylamine and n-propanol).

Preferably, the organic solvent used is toluene and the reaction is carried out between 25 and 65°C, preferably at 45°C.

In the process of enzymatic resolution, the nucleophile used is n-propanol, in a ratio of n-propanol to the starting compound (I) between 1 and 2 equivalents. The n-propanol has a variety of unexpected advantages over the preceding technology disclosed in US-6969767, ES-2203319:
◆ it shows a high reaction rate (16-24 h) compared to the previous processes (100 h)
◆ it provides a high stereoselectivity: ee>99,0%
◆ the reaction subproducts are liquid, allowing them to be removed with the mother liquors from crystallization and then to wash the chiral carbonate of formula (S)-(I).

In the process triethylamine is used as an additive in a ratio between 0,5 and 3 equivalents with regard to the starting product (I).

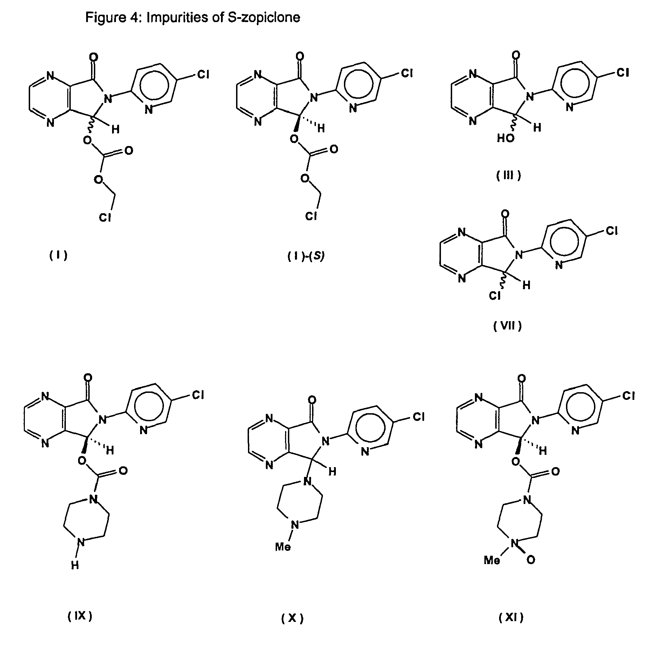

The reaction medium, toluene/n-propanol/triethylamine, has a variety of unexpected advantages over the preceding technology disclosed in US-6969767, ES-2203319:
◆ it enables a reaction concentration to be achieved so that the process is suitable for the industrial development. The reaction concentration is placed between 10 and 20 ml of solvent per gram of starting intermediate (preferred concentration = 14 ml/g), instead of the previous concentration of 200 ml of solvent per gram of starting intermediate.
◆ The product of the alcoholysis reaction, i.e. the alcohol of formula (III) in figure 3, has a very low solubility in that medium. This allows the product to be separated from reaction medium and later to be recycled as a starting material of (S)-zopiclone
◆ It enables the lipase to be re-used directly for the further cycles.
◆ In these conditions the competition reaction with formation of the chloro-impurity, i.e. the 6-(5-chloropyrid-2-il)-5-chloro-7-oxo-5,6-dihydropyrrolo[3,4-b]pyrazine of formula (VII) in figure 4, is reduced to a minimum.

The starting intermediate is dissolved in the mixture toluene/n-propanol/triethylamine, and then an amount of enzyme of lipase type of fraction B of *Candida antarctica* immobilized on a macroporous resin (preferably Novozyme^{®} 435) is added. This amount is placed between 0,25 and 1 g of commercial lipase per gram of starting intermediate of formula (I) in figure 3.

The reaction is followed up by means of HPLC under these conditions:
◆ mobile phase: hexane:ethanol 0,25 % DEA (60:40)
◆ stationary phase: Chiralpak AS 25x0,46 cm
◆ flow: 0,7 ml/min
◆ detection: 303 nm
◆ temperature: 30°C
◆ injection volume: 10 µl,
◆ sample concentration: approx. 0,25 mg/ml
till an enantiomeric excess higher than 98,0% (preferably >99,0%) is reached.

After reaching the desired enantiomeric excess, the reaction mixture is filtered through a double filter, which on a first surface has a metallic sieve of 250 micron, which allows the removal of the enzyme, and on a second surface has a paper filter, which allows to separate the alcohol of formula (III) from chiral carbonate (S)-(I), the precursor of (S)-zopiclone. This intermediate alcohol can be used without further purification in the preparation of starting racemic carbonate of formula (I).

The filtered enzyme then can be re-used in a new process.

The reaction mixture, which contains the chiral carbonate (S)-(I), not reacted in the alcoholysis process, is concentrated under vacuum to remove toluene, till a fluid paste is formed, to which isopropanol is added in order to crystallize the reaction product.

Filtration, washing with isopropanol and then drying under vacuum (40-300 torr) and at a temp. (25-40°C) provide a product, which is suitable for the following reaction step. Some of its properties are listed in the following table.

| ee | % HPLC in area | | |
|---|---|---|---|
| | % (III) | % (VII) | % (S)-(I) |
| >98,5 % | <5 % | <1 % | 89-95 % |

In another aspect of this invention the optically enriched carbonate of formula (S)-(I) is transformed into (S)-zopiclone of formula (S)-(II) by reaction with N-methylpiperazine in an inert organic solvent, miscible with water, for instance acetonitrile, tetrahydrofurane, dioxane or acetone, among others. It's recommendable to use a ratio of N-methylpiperazine higher than the equimolar one with regard to carbonate (S)-(I), for instance from 1 to 3, or even higher. Preferably, the reaction is carried out at a temperature under 15°C. The (S)-zopiclone thus obtained can be purified by means of crystallization or chromatography.

In another aspect of this invention, the so obtained (S)-5-(chloromethyloxycarbonyloxy)-6-(5-chloropyrid-2-il)-7-oxo-5,6-dihydropyrrolo[3,4b]pyrazine of formula (S)-(I) is used as a starting material in the preparation of (S)-zopiclone (S)-(II), by means of its reaction with an excess of N-methylpiperazine in an organic solvent, immiscible with water, preferably the methylene chloride. Once the reaction is completed, the reaction mixture is washed with purified water. The organic phase is concentrated under vacuum and the (S)-zopiclone is crystallized by the addition of isopropanol. The product is isolated by filtration and dried under vacuum and temperature.

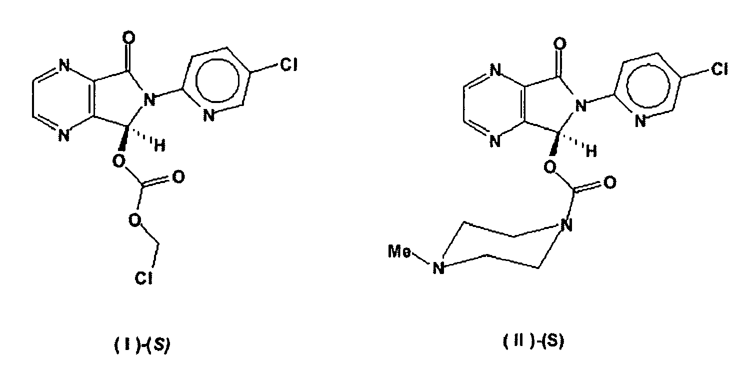

The introduction of methylene chloride as a reaction solvent may be considered as trivial, but it is not, because it has a number of not obvious, but important advantages from the point of view of the industrial practice.
◆ It allows the process to be carried out in an homogeneous phase at a higher concentration, because the reaction product has a high solubility in methylene chloride.
◆ It allows the removal of excess N-methylpiperazine and reaction subproducts by washing with purified water. This operation allows the (S)-zopiclone to be isolated from reaction medium in the absence of N-methylpiperazine and therewith it reduces to a minimum the presence of synthetic impurities, e.g. the decarbonyloxyzopiclone of formula (X) in figure 4, the formation of which is favoured by the heating of (S)zopiclone in the presence of N-methylpiperazine during the process of solvents evaporation.
◆ It enables an anhydrous crystallization process to be developed through concentration under vacuum and the addition of isopropanol.

This synthetic procedure for (S)-zopiclone yields a product with high purity, some of which properties are included in the following table.

| yield | ee | % HPLC in area | | | |
|---|---|---|---|---|---|
| | | % (III) | % (S) (I) | % (X) | % (S) (II) |
| 80-90% | >98,5% | <5% | <2% | <1% | 92-97% |

The processes, which are the object of the present invention, have been designed for the practice on an industrial scale, avoiding the limitations of preceding processes.

The (Z)-zopiclone obtained according to the processes, which are object of the present invention, has a high degree of purity, which allows its use as a starting material for the formulation of drugs to be sold in markets ruled by GMP quality criteria. In this context, the (S)-zopiclone must have a purity of >99,0 % ee, must be free of unknown impurities, i.e. <0,1 % and must have a minimal number of known impurities and also a minimal percentage of these.

The high insolubility of some impurities, e.g. the alcohol of formula (III), makes the purification of (S)-zopiclone by column chromatography recommendable. The selected stationary phase is silica gel SiO₂ 63-200 µm. The process of this invention is perfectly scalable up to the conditions of the industrial practice. The disclosed example of the present invention is carried out in a stainless steel column, with 100 mm diameter. The procedure is as follows:
◆ dry filling with 1350 g of SiO₂ 63-200 µm at a pressure of 15-20 bar, conditionned through recirculation of acetone for 5 h;
◆ column balancing with acetone or with triethylamine/ acetone between 2 and 5 %;
◆ loading of 100 g of (S)-zopiclone dissolved in dichloromethane;
◆ chromatography with ethyl acetate or acetone with a flow of 500 ml/min, removing the first chromatographic band, which includes the most impurities;
◆ chromatography with acetone at 500 ml/min, collecting the main fraction, which contains the (S)-zopiclone;
◆ column washing with acetone, which has a triethylamine content between 2 and 5%, in order to remove the impurities retained in the column (essentially the impurity of formula X).

The subsequent isolation by concentration of the fraction enriched in (S)-zopiclone under vacuum and the crystallization from isopropanol afford an anhydrous crystalline product, whose main residual solvents belong to class 3 according to the Guideline for Residual Solvents ICH Q3C.

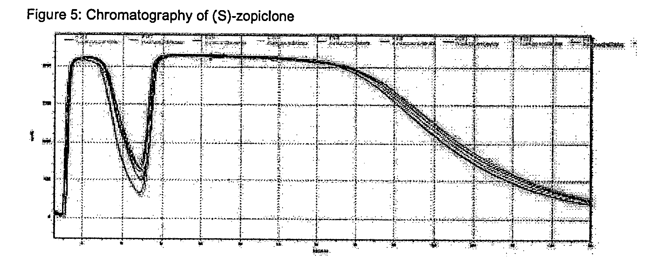

After conditioning with acetone, the column is used for successive separations, with an excellent reproducibility which allows the process to be validated from the GMP point of view. Figure 5 shows the chromatographic front lines of a series of purifications, which were performed using this regeneration procedure for the chromatographic support.

The chromatographic fraction, which contains the (S)-zopiclone, is treated as follows:
a) filtration
b) concentration of this solution under vacuum, till nucleation
c) addition of isopropanol and partial concentration under vacuum
d) solid separation from liquid by filtration or centrifugation
e) drying under a vacuum between 40 and 400 torr and a temperature between 20 and 40°C.

In this way a product is obtained, which has a high purity, and whose main specifications are listed in the following table:

| Assay | | Result |
|---|---|---|
| 1. | aspect | white solid, free from visible impurities |
| 2. | solution aspect | |
| | colour | <Yₛ |
| | haze | <B₁ |
| 3. | specific rotation | 135° |
| 4. | impurities (HPLC) | |
| | XI¹ | 0,01 % |
| | III¹ | 0,06 % |
| | L¹ | ND |
| | IX¹ | ND |
| | VII¹ | ND |
| | X¹ | 0,05 % |
| | main known impurity | 0,01 % |
| | impurities total: | 0,13 % |
| 5. | richness (assessed) | 99,7 % |
| 6. | enantiomeric excess | 100 % |
| 7. | residual solvents | |
| | isopropanol | <0,1 % |
| | toluene | 0,9 ppm |
| | methylene chloride | ND |
| | ethyl acetate | ND |
| | n-propanol | ND |
| | acetone | <0,1 % |
| | triethylamine | <0,1 % |

| | | |
|---|---|---|
| ¹ see figure 4 | | |

Applying the procedure of the present invention an anhydrous crystalline (S)-zopiclone is obtained, which has been thermically studied by differential scanning calorimetry (DSC) and thermogravimetry (TG).

Both TG and DSC are obtained with a heating rate of 10°C/minute, with a N₂ flow 200 ml/minute. Starting at room temperature, the DSC is measured up to 300°C, whereas for the TG the temperature reached 800°C.

In the DSC a strong endothermic reaction is observed at about 205°C, and a low exothermic reaction with a maximum at about 270°C. Furthermore, it appears a sole absorption peak, suggesting that only one phase exists (the peak is so narrow, that although a second phase could have a very next endothermic reaction, they would not overlap completely and a pronounced asymmetry would be seen in the peak).

In TG one can observe that up to 250°C there is no mass loss, but above that temperature the sample decomposition starts with a quick mass loss.

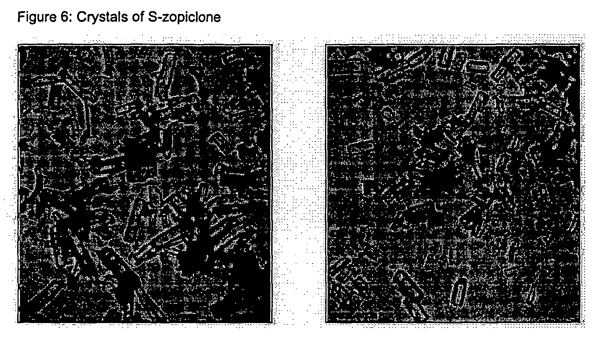

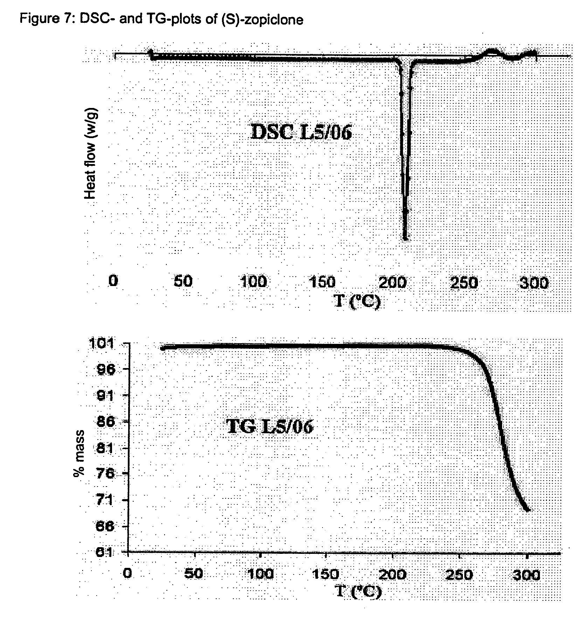

The conclusion is clear: the procedure of the present invention allows an anhydrous crystalline product to be prepared and such a product shows only one polymorph. The graphic results of these measurements are shown in figures 6 and 7.

The microscope analysis and the X ray diffraction in a NOVA-Xcalibur diffractometer, which reduces to a minimum the preferred orientation effects, allow to conclude that the according to the procedure of this invention prepared (S)-zopiclone is free from noticeable non crystalline phase and it crystallizes in rhombic system with spatial group P2₁2₁2₁ and following values of crystal cell:
a = 5,567(3) Å
b = 8,852(2) Å
c = 35,677(17) Å
V = 1758,13 Å³

Figure 8 shows a comparison between the obtained experimental profile, the theoretical calculated profile and the adjustment Rietveld of such mentioned experimental profile. The results are considered as satisfactory both graphically and numerically during the characterization of according the procedure of present invention prepared product, that is, the known anhydrous crystalline polymorph of (S)-zopiclone.

The present invention is described by the following non-limiting examples.

### Examples

### Example 1

### Preparation of (S)-5-(chloromethyloxycarbonyloxy)-6-(5-chloropyrid-2-il)-7-oxo-5,6-dihydropyrrolo{3,4b]pyrazine

In a reactor containing 3500 ml of toluene, 250 g of 5-(chloromethyloxycarbonyloxy)-6-(5-chloropyrid-2-yl)-7-oxo-5,6-dihydropyrrolo[3,4b]pyrazine are loaded and it is heated to 45°C until it dissolves. Then 58,9 ml of triethylamine and 79 ml of n-propanol are added, followed by 125 g of Novozyme^{®} 435. The mixture is stirred at 45°C for 16-24 h a 45°C and the enzyme and the precipitated product are separated by selective filtration. The organic phase is concentrated under vacuum, till a fluid paste is obtained, 800 ml of isopropanol are added, the mixture is cooled to 0°C. The product is then filtered and dried under vacuum to give between 96 and 111 g of (S)-5-(chloromethyloxycarbonyloxy)-6-(5-chloropyrid-2-yl)-7-oxo-5,6-dihydropyrrolo[3,4b]pyrazine, characterized by the following data:
white solid
[α]_{D}²⁰ = +94,5 (c = 1,1, CHCl₃, ee>99%)
R_{f} (SiO₂, ethyl acetate/acetone 1/1) = 0,80
t_{R} [C18 5 µm, 150 x 4,6mm, (0,16% NaH₂PO₄ +0,81%SDS, pH = 3,5)/MeCN = 62/38, 1,3 ml/min, 30°C, 303 nm, 0,5 mg/ml, 20 µl] = 14,7 min
t_{R} [Chiralpak AS, 250 x 4,6mm, (0,25% Et₂NH in EtOH) / hexane = 40/60, 0,7 ml/min, 30°C, 303 nm, 0,25 mg/ml, 10 µl] = 13,4 min (R-enantiomer at 16,2min)
melting point: 135-137°C
IR (KBr, cm⁻¹) = 3065, 1783, 1741, 1578, 1466, 1378, 1243, 1146, 1095
¹H-RMN (CDCl₃, 300 MHz) δ (ppm) = 8,92 (d, J = 2,5 Hz; 1H), 8,84 (d, J = 2,5 Hz; 1H), 8,50 (d, J = 8,9 Hz; 1H), 8,37 (d, J = 2,6 Hz; 1H), 7,97 (s, 1H), 7,79 (dd, J = 8,9, 2,6 Hz; 1H), 5,88 (d, J = 6,3 Hz; 1H), 5,73 (d, J = 6,3 Hz; 1H)
¹³C-RMN (CDCl₃, 75 MHz) δ (ppm) = 162,4 (CO), 153,9 (CO), 152,1 (C), 148,5 (CH), 148,4 (CH), 147,4 (C), 146,8 (CH), 144,0 (C), 138,3 (CH), 128,7 (C), 115,7 (CH), 80,9 (CH), 72,6 (CH₂)
MS (ESI⁺, m/z) = 355 [(M+H)⁺, 100%]

### Example 2

### Preparation of (S)-zopiclone

To a solution of 150 g of (S)-5-(chloromethyloxycarbonyloxy)-6-(5-chloropyrid-2-il)-7-oxo-5,6-dihydropyrrolo-[3,4b]pyrazine in 1725 ml of methylene chloride, under inert gas and at 0°C 150 ml of N-methylpiperazine are added slowly. Once the addition is finished, the mixture is allowed to warm up to 15°C. After 1-2 h at this temperature, the mixture is cooled again to 0°C and 900 ml of purified water are added while keeping the temperature under 5°C. The aqueous phase is separated and the organic phase is concentrated under vacuum. The addition of 750 ml of isopropanol, followed by cooling to 0°C allow to obtain, after filtration and drying under vacuum, between 140 and 145 g of (S)-zopiclone, characterized by the following data:
white solid
melting point: 176-1781C
[α]_{D}²⁰ = 135°
ee>99%)
R_{f} (SiO₂, ethyl acetate/acetone 1/1) = 0,15
t_{R} [C18 5 µm, 150 x 4,6mm, (0,16% NaH₂PO₄ +0,81%SDS, pH = 3,5)/MeCN = 62/38, 1,3 ml/min, 30°C, 303 nm, 4,0 mg/ml, 20 µl] = 26,9 min
IR (KBr, cm⁻¹) = 2942, 2790, 1730, 1715, 1463, 1372, 1140, 1087
¹H-RMN (CDCl₃, 300 MHz) δ (ppm) = 8,88 (d, J = 2,6 Hz; 1H), 8,84 (d, J = 2,6 Hz; 1H), 8,49 (d, J = 8,9 Hz; 1H), 8,37 (d, J = 2,6 Hz; 1H), 8,00 (s, 1H), 7,78 (dd, J = 8,9, 2,6 Hz; 1H), 3,80-3,60 (m br; 2H), 3,60-3,40 (m br, 2H), 2,50-2,30 (m br, 2H), 2,24 (s, 3H), 2,30-2,00 (m br; 2H)
¹³C-RMN (CDCl₃, 75 MHz) δ (ppm) = 162,8 (CO), 155,5 (CO), 153,3 (C), 148,3 (CH), 147,7 (CH), 147,7 (C), 146,6 (CH), 143,8 (C), 138,0 (CH), 128,2 (C), 116,0 (CH), 79,0 (CH), 54, 4 (2 CH₂), 46,0 (CH₃), 43, 9 (2 CH₂)
MS (ESI⁺, m/z) = 389 [(M+H)⁺, 65%], 411 [(M+Na)⁺, 100%]

### Example 3

### Chromatographic purification and isolation of (S)-zopiclone

The isolation of the present invention is carried out in a stainless steel column of 100 mm diameter. The procedure is as follows: the column is dry filled with 1350 g of SiO₂ 63-200 µm at a pressure of 15-20 bar and acetone is added by recirculation for 5 h.

The column is balanced with 10 1 of acetone and is loaded with 100 g of (S)-zopiclone, prepared according to the example 2 method, dissolved in 500 ml of methylene chloride. Eluting with ethyl acetate or acetone at a flow rate of 500 ml/min and collecting of the first chromatographic band, which contains the most impurities. The collector tubes are substituted by new ones, then elution with acetone at a flow rate of 500 ml/min and collection of the main fraction, which contains (S)-zopiclone (total 17-18 1). The column is washed with 13 1 of acetone with 2% triethylamine in order to remove impurities, which are retained in the column (essentially the impurity of formula X).

The fraction containing pure (S)-zopiclone is concentrated under vacuum, until almost no acetone is left in the supernatant liquor on the solid, 400 ml of isopropanol are added and acetone is removed under vacuum. The resulting suspension is cooled to 0°C, filtered, washed with isopropanol (3x25 ml) cooled to 0°C and dried under vacuum to give 80-85 g of (S)-zopiclone, with following analytical results:

| Assay | | Result |
|---|---|---|
| 1. | aspect | white solid, free from visible impurities |
| 2. | solution aspect | |
| | colour | <Yₛ |
| | haze | <B₁ |
| 3. | specific rotation | 135° |
| 4. | impurities (HPLC) | |
| | XI¹ | 0,01 % |
| | III¹ | 0,06 % |
| | L¹ | ND |
| | IX¹ | ND |
| | VII¹ | ND |
| | X¹ | 0,05 % |
| | main known impurity | 0,01 % |
| | impurities total: | 0,13 % |
| 5. | richness (assessed) | 99,7 % |
| 6. | enantiomeric excess | 100 % |
| 7. | residual solvents | |
| | isopropanol | <0,1 % |
| | toluene | 0,9 ppm |
| | methylene chloride | ND |
| | ethyl acetate | ND |
| | n-propanol | ND |
| | acetone | <0,1 % |
| | triethylamine | <0,1 % |

| | | |
|---|---|---|
| ¹ see figure 4 | | |

## Claims

1. A process for the preparation of enantiomerically pure {S)-5-{chloromethyloxycarbonyloxy)-6-{5-chloropyrid-2-yl)-7-oxo-5,6-dihydropyrrolo[3,4b]pyrazine (S)-(I) according to following scheme, **characterized in**: 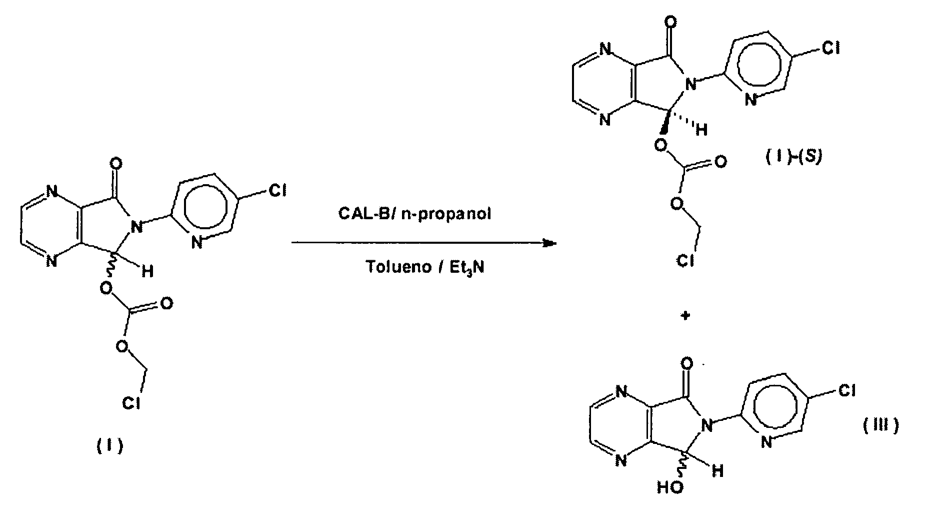
a) stereospecific alcoholysis of an enantiomeric mixture of a compound of formula (I) in the presence of an enzyme and an aliphatic amine in an organic solvent
b) separation of enzyme and hydrolysis "in situ" racemizated product (III) by simple filtration
c) isolation and purification of non-reactive chiral carbonate (I)-(S) by crystallization.

2. A process according to claim 1 **characterized in that** the enantiomeric mixture is a racemic mixture.

3. A process according to claim 1 **characterized in that** the enantiomeric mixture is enriched in enantiomer (S).

4. A process according to claim 1 **characterized in that** the organic solvent used is toluene.

5. A process according to claim 1 **characterized in that** the reaction is carried out between 25 and 65°C.

6. A process according to claim 1 **characterized in that** the reaction concentration is placed between 10 and 20 ml of solvent per gram of starting intermediate.

7. A process according to claim 1 **characterized in that** the reaction concentration is 14 ml of solvent per gram of starting intermediate (I).

8. A process according to claim 1 **characterized in that** the nucleophile alcohol is n-propanol.

9. A process according to claim 8 **characterized in that** the ratio of n-propanol is placed between 1 and 2 equivalents with regard to the starting product (I).

10. A process according to claim 1 **characterized in that** the aliphatic amine is triethylamine.

11. A process according to claim 10 **characterized in that** the ration of triethylamine is placed between 0,5 and 3 equivalents with regard to the starting product (I).

12. A process according to claim 1 **characterized in that** the reaction is catalized by an enzyme of lipase type.

13. A process according to claim 12 **characterized in that** the lipase is the B-fraction of *Candida antarctica.*

14. A process according to claim 12 **characterized in that** the lipase is immobilized on a macroporous resin.

15. A process according to claim 14 **characterized in that** the lipase is Novozyme^{®} 435.

16. A process according to claims 12 to 15 **characterized in that** the ratio of used lipase is placed between 0,25 and 1 equivalent with regard to the starting product (I)

17. A process according to claims 12 to 15 **characterized in that** the lipase can be reused in a further reaction.

18. A process according to claims 12 to 15 **characterized in that** the alcoholysis product (III) and the lipase are recovered by filtration.

19. A process according to claim 18 **characterized in that** the alcoholysis product (III) and the lipase are separated by selective filtration.

20. A process according to claim 1 **characterized in that** the solvent for the crystallization of obtained chiral carbonate (S)-(I) is isopropanol.

21. A process for the preparation of optically pure (S)-zopiclone (S)-(II) consisting in the reaction of an excess of N-methylpiperazine in an organic solvent with the optically pure carbonate of formula (S)-(I), which in turn was obtained according to the chemoenzimatic resolution process described in claims 1 to 20.

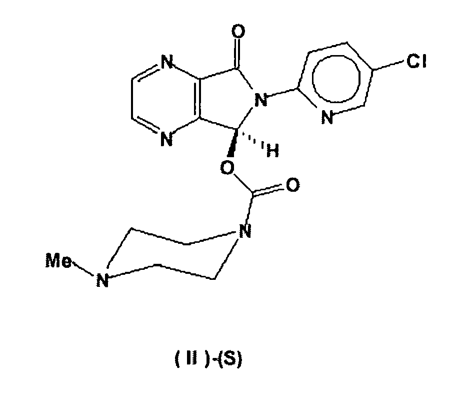

22. A process for the preparation of optically pure (S)-zopiclone according to claim 21 **characterized in that** the solvent is methylene chloride.

23. A process for the preparation of optically pure (S)-zopiclone according to claim 21 **characterized in** the removal of excess of N-methylpiperazine from reaction medium before the isolation of (S)-zopiclone is done.

24. A process of purification of (S)-zopiclone by non-chiral column chromatography.

25. A process according to claim 24, **characterized in that** the stationary phase is silica gel SiO₂ 63-200 µm.

26. A process according to claim 24 **characterized in that** the mobile phase consists of methylene chloride, ethyl acetate, acetone and triethylamine or a mixture thereof.

27. A process according to claim 24 **characterized in that** the obtained (S)-zopiclone has an individual content of impurities under 0,1 %.

28. A process according to claim 24 **characterized in that** the obtained (S)-zopiclone has an enantiomeric excess higher than 99,0 %.

29. A process of crystallization of anhydrous crystalline (S)-zopiclone **characterized by**:
f) filtration of a solution of (S)-zopiclone in acetone
g) concentration of this solution under vacuum till nucleation
h) addition of isopropanol
i) solid-liquid separation by filtration or centrifugation
j) drying under a vacuum between 40 and 400 torr and at a temperature between 20 and 40°C.

30. A process according to claim 29 **characterized in that** a polymorph of (S)-zopiclone is obtained, which crystallizes in the rhombic system with the following values for its crystal cell:
a = 5, 567 (3) Å
b = 8,852(2) Å
c = 35,677(17) Å
V = 1758,13 Å³
